# EUROPEAN PATENT SPECIFICATION

(11) **EP 3 572 079 B1**
(45) Date of publication and mention of the grant of the patent: **08.09.2021**
(21) Application number: 18382389.7
(22) Date of filing: 04.06.2018
(51) Int. Cl.: A61K 31/185, A61P 31/22

(54) **ETAMSYLATE FOR TREATMENT OF HERPES VIRUS INFECTIONS**
ETAMSYLAT ZUR BEHANDLUNG VON HERPESVIRUSINFEKTIONEN
ETAMSYLATE POUR LE TRAITEMENT D'INFECTIONS PAR LE VIRUS DE L'HERPÈS

(30) Priority: 22.05.2018 ES 201830489
(43) Date of publication of application: 27.11.2019
(73) Proprietor: Dobecure, S.L., 28017 Madrid (ES)
(72) Inventor: Cuevas Sánchez, Pedro, 28035 Madrid (ES); Giménez Gallego, Guillermo, 28015 Madrid (ES)
(74) Representative: Monzón de la Flor, Luis Miguel

(56) References cited:
- WO-A1-2016/090362
- WO-A2-01/13910
- MANUELA DONALISIO ET AL: "Inhibition of Human Respiratory Syncytial Virus Infectivity by a Dendrimeric Heparan Sulfate-Binding Peptide", ANTIMICROBIAL AGENTS AND CHEMOTHERAPY, vol. 56, no. 10, 30 July 2012 (2012-07-30) , pages 5278-5288, XP055548367, US ISSN: 0066-4804, DOI: 10.1128/AAC.00771-12

## Description

### FIELD OF THE INVENTION

The invention describes etamsylate for use in the treatment of virus infections using heparan sulfate for cell penetration, wherein the virus infection is a herpes simplex virus infection occurring in mucosal/epithelial surfaces such as genital and herpetic stromal keratitis, particularly for the treatment of herpes labialis condition.

The etamsylate as therapeutic agent can be used as a pharmaceutically acceptable salt or solvate thereof.

### BACKGROUND OF THE INVENTION

Herpes simplex virus-1 (HSV-1) causes lifelong infection affecting between 50-90% of the global population. In addition to causing dermal lesions, HSV-1 is a leading cause of blindness resulting from recurrent corneal infection (Vet Microbiol 2002, 86: 17). The major ocular manifestations of herpetic infection are keratitis, blepharitis, conjunctivitis, uveitis, dry eye and retinitis (Arq Bras Ofhalmol 2013; 17: 129-132). HSV-1 is a neurotropic member of the alpha herpesvirus family. Primary infection typically occurs following inoculation of mucosal epithelial surfaces, using heparan sulfate for cell penetration. During initial infection virions gain access to sensory nerve fibers and are transported to neural cell bodies in the trigeminal ganglia where HSV-1 establishes a latent infection (J Clin Microbiol 2003, 41: 84; Sex Transm Infect 2005; 81: 103). HSV-1 is the primary cause of facial lesions (mouth, lips and eyes) in humans.

Management of HSV-1 infections usually depends on a combination of antiviral and anti-inflammatory drugs, such as corticoids with the latter often needed for lengthy periods, which can result in unwanted side effects. In addition, the widespread use of acyclovir and nucleosides analogs has led to emergence of HSV-1 strains that are resistant to these drugs. As such, there is an urgent need for alternative and more effective therapies to control HSV-1 infections.

In the state of the art are known the following documents:
- WO 01/13910 A2 discloses polysaccharide preparations enriched in 3-OST-3 modified heparan sulfate. Also disclosed are methods of treating herpes simplex viral type-1 infection using the pharmaceutical preparations of the invention.
- WO 2016/090362 A1 discloses a method of identifying a promising cellular antiviral or bacterial toxin drug target.

Inventors have surprisingly found that local administration of etamsylate in virus infections using heparan sulfate for cell penetration shows clinical benefits, it also happens with virus infections occurring in mucosla/epithelial surfaces and particularly in herpes liabilis condition.

### DESCRIPTION OF THE INVENTION

The invention is defined by the claims. Any subject-matter falling outside the scope of the claims is provided for information purposes only. Local administration of the compound can be in the form of creams, gels, ointments, dispersions, solid sticks, emulsions and micro-emulsions that can be formulated according to the conventional methods using suitable excipients. Acceptable vehicles and solvents that can be used are water, Ringer's solution, and isotonic sodium chloride solution.

Generally, the dosage required providing an effective amount of the compound and composition, which can be adjusted by one of ordinary skill in the art, will vary depending on the extent of the disease and frequency of treatment.

The object of the present invention is directed to etamsylate for use in the treatment of a viral infection as described in the appended claims. The present invention may be used for the treatment or prevention of herpes labialis and other viral infections using heparan sulfate for cell penetration as described in the appended claims. As used throughout the disclosure, the following terms, unless otherwise indicated, shall be understood to have the following meanings.

"Patient" refers to human and includes males and females.

"Treating", "treat" or "treatment" refers to using the compound of the present invention either prophylactically to prevent the symptoms of the disease, or therapeutically to ameliorate an existing condition.

"Therapeutic agent" includes any therapeutic agent which can be used to treat or prevent the disease described herein. Therapeutic agent includes but is not limited to antiviral, anti-inflammatory compounds and the like. Therapeutic agent includes the pharmaceutically acceptable salts.

Except when indicated otherwise, all of the technical and scientific elements used in this specification have the meaning commonly understood by a person with average skill in the art to which this invention belongs.

Throughout the description and claims, the word "comprise" and its variants are not intended to exclude other technical characteristics, additives, components or steps.

### EXAMPLE OF THE INVENTION

The following non-limiting example describes the use of the present invention.

### Efficacy of etamsylate in herpes labialis condition

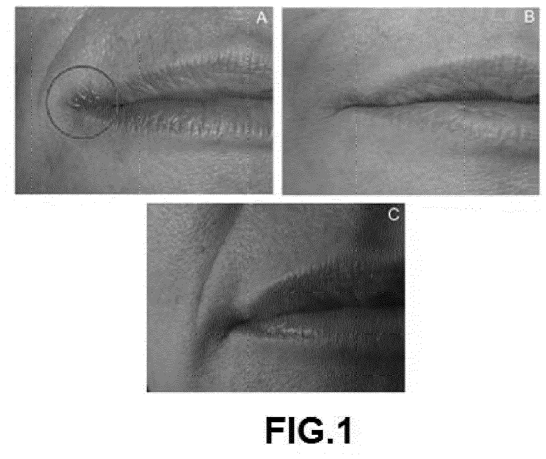

A 44-years old woman presented with a herpes labialis lesion in her right labial commissure (Figure1). After discussing the various treatment options, the patient opted to try topically administration of etamsylate and signed an informed consent. Patient self-administered etamsylate (OM Pharma Switzerland) in a 12% glycerin solution three times daily until day 4. After one day of treatment patient was free of pain and after four days of treatment herpetic lesion healed without any cosmetic apparent disturbance (Figure 1). She reported no recurrent episodes of herpetic lesion in her right labial commissure after several months of treatment. Although the action mechanism of etamsylate remains unknown, we suggest that this drug acts blocking heparan sulfate and consequently impedes virus penetration into the cytoplasm.

### EXPLANATION OF THE DRAWINGS

As a complement to the present description, and for the purpose of helping to make the characteristics of the invention more readily understandable, in accordance with a preferred practical exemplary embodiment thereof, said description is accompanied by a set of drawings constituting an integral part of the same, which by way of illustration and not limitation represent the following. Figure 1. Shows the Effects of etamsylate in herpes labialis

Appearance of herpes labialis lesion treated by local application of etamsylate at baseline (A), at two days (B) and at four days (C). Red circle indicates right labial commissure, where the herpes liabilis appeared and in state (B) and (C) can be observed its evolution.

## Claims

1. Etamsylate as therapeutic agent or a pharmaceutically acceptable salt or solvate thereof for use in the treatment of virus infection using heparan sulfate for cell penetration **characterized in that** the virus infection is herpes simplex virus infection occurring in mucosal/epithelial surfaces such as genital and herpetic stromal keratitis.

2. Etamsylate as therapeutic agent or a pharmaceutically acceptable salt or solvate thereof for use in the treatment of virus infection using heparan sulfate for cell penetration **characterized in that** the virus infection is herpes labialis.

## Patentansprüche

1. Etamsylat als therapeutisches Mittel oder ein pharmazeutisch verträgliches Salz oder Solvat davon zur Verwendung bei der Behandlung von Virusinfektionen unter Verwendung von Heparansulfat zur Zellpenetration, **dadurch gekennzeichnet, dass** die Virusinfektion eine Herpessimplex-Virusinfektion ist, die an Schleimhaut-/Epitheloberflächen, wie z. B. Genitalien und herpetische stromale Keratitis, auftritt.

2. Etamsylat als therapeutisches Mittel oder ein pharmazeutisch verträgliches Salz oder Solvat davon zur Verwendung bei der Behandlung von Virusinfektionen unter Verwendung von Heparansulfat zur Zellpenetration, **dadurch gekennzeichnet, dass** die Virusinfektion Herpes labialis ist.

## Revendications

1. Etamsylate en tant qu'agent thérapeutique ou un sel ou solvate pharmaceutiquement acceptable de celui-ci à utiliser dans le traitement d'une infection virale utilisant du sulfate d'héparane pour pénétration cellulaire **caractérisée en ce que** l'infection virale est une infection par le virus herpès simplex survenant dans les surfaces muqueuses / épithéliales telles que la kératite stromale génitale et herpétique.

2. Etamsylate en tant qu'agent thérapeutique ou un sel ou solvate pharmaceutiquement acceptable de celui-ci à utiliser dans le traitement d'une infection virale utilisant du sulfate d'héparane pour la pénétration cellulaire **caractérisée en ce que** l'infection virale est l'herpès labial.
